# EUROPEAN PATENT APPLICATION

(11) **EP 4 039 827 A1**
(43) Date of publication of application: **10.08.2022**
(21) Application number: 20871183.8
(22) Date of filing: 29.09.2020
(51) Int. Cl.: C12Q 1/6883, G01N 33/68

(54) **BIOMARKER COMPOSITION FOR PREDICTING THERAPEUTIC EFFECT OF MESENCHYMAL STEM CELL ON RENAL FIBROSIS**

(30) Priority: 02.10.2019 KR 20190122192
(71) Applicant: Corestem Co., Ltd., Seongnam-si, Gyeonggi-do 13486 (KR); Chungbuk National University Industry-Academic Cooperation Foundation, Cheongju-si, Chungcheongbuk-do 28644 (KR)
(72) Inventor: KIM, Kyung Suk, Seoul 02838 (KR); HAN, Sang Bae, Cheongju-si, Chungcheongbuk-do 28692 (KR); LEE, Tae Yong, Yongin-si, Gyeonggi-do 17128 (KR); KIM, Hyung Sook, Cheongju-si, Chungcheongbuk-do 28109 (KR)
(74) Representative: HGF
(86) International application number: PCT/KR2020/013353
(87) International publication number: WO 2021/066527

(57) **Abstract**

The present invention relates to a biomarker composition for predicting the therapeutic effect of mesenchymal stem cells on renal fibrosis.

## Description

### Technical Field

This application claims priority benefit to Korean Patent Application No. 10-2019-0122192 filed on October 2, 2019, which is incorporated herein by reference in its entirety.

The present invention relates to a biomarker composition for predicting the therapeutic effect of mesenchymal stem cells on renal fibrosis.

### Background Art

Fibrosis is a disease characterized by excessive deposition of connective tissue proteins involving abnormal extracellular matrix metabolism in organs such as lung, heart, liver, and kidney. Fibrosis can lead to a decrease in healthy cells in any organ or tissue, and an increase in the mass of fibrotic connective tissue, which in turn can damage the normal structure of the organ or tissue. Such damage can impair the physiological and biochemical functions of the affected organ or tissue, and can cause complete failure of the organ. Diagnostic methods, prevention and treatment methods for organ and tissue fibrosis have been extensively studied. Although the pathogenesis of fibrosis cannot be fully elucidated at the present time, in general, fibrosis of an organ or tissue is caused by multiple factors, such as inflammation, immunological response, ischemia, hemodynamic changes, and the like. Accordingly, damaged soft tissue cells activate macrophages to release a number of cytokines and growth factors.

Chronic kidney disease refers to kidney damage or continuous decline in kidney function for more than 3 months. Fibrosis of kidney tissue (renal fibrosis) is a common phenomenon seen in all kidney diseases that progress to chronic kidney disease. Fibrosis is the hardening and formation of scar tissue, and is known to be caused by cytokines, immune cells, reactive oxygen species, fibroblast growth factors, and proteolytic regulation enzymes that cause inflammation. In chronic kidney disease, extensive scar tissue resulting from renal fibrosis is commonly observed, and is directly related to decreased renal function and prognosis.

Mesenchymal stem cells are highly proliferative adherent cells having multipotency capable of differentiating into bone, cartilage, fat and the like, and are known to have antiinflammatory and immunomodulatory abilities. Mesenchymal stem cells exhibit immunosuppressive effects such as inhibition of proliferation and differentiation of T cells and B cells, and inhibition of functions of immune cells such as dendritic cells, natural killer (NK) cells and macrophages. Recently, a study of increasing the engraftment rate of hematopoietic stem cells by transplanting mesenchymal stem cells together with hematopoietic stem cells has been reported. It has been reported that mesenchymal stem cells reduce inflammation and inhibit autoimmune hyperreactivity in diseases such as graft versus host disease (GVHD), collagen-induced arthritis (CIA), experimental autoimmune encephalomyelitis (EAE), sepsis, acute pancreatitis (AP), colitis, multiple sclerosis (MS) and rheumatoid arthritis. Recently, there is also a report that mesenchymal stem cells administered intraarterially prevent renal fibrosis.

Previously, in order to confirm the therapeutic effect of mesenchymal stem cells in renal fibrosis disease animal models, survival rate, the appearance of the kidney, the weight of the kidney, proteinuria and creatinine in blood were measured. However, the existing biomarkers for predicting the therapeutic effect have limitations in that the measurement thereof is difficult, it can be measured after the disease has progressed considerably, and the accuracy is low. Therefore, there is a need for research on new biomarkers.

### Detailed Description of Invention

### Technical Problem

The present inventors measured the therapeutic effect of mesenchymal stem cells in a renal fibrosis disease animal model using the existing biomarkers (survival rate, the appearance of the kidney, the weight of the kidney, proteinuria and creatinine in blood, the size of the glomerulus, glomerular sclerosis level, and tubular damage), and confirmed that new biomarkers (chemokines and CHI3L1) may be also measured. Based on the above, the present inventors completed the present invention.

More specifically, it was confirmed that administration of adriamycin leads to renal fibrosis in mice, which leads to renal fibrosis, turns hard and white, increases in weight, increases proteinuria levels, increases blood creatinine concentration, increases renal fibrosis, and increases the expression of EMT markers and inflammatory cytokines, and the glomerulus is hardened due to increased collagen production, and the tubules are contracted and eventually leads to death. When mesenchymal stem cells were administered to these mice, these mice showed a similar renal status to that of the control group in which renal fibrosis was not induced, decreased kidney weight, decreased proteinuria level and blood creatinine concentration, decreased renal fibrosis, the decreased expression of EMT markers and inflammatory cytokines, and increased VEGF. Histopathologically, it was confirmed that collagen production in the glomerulus was decreased, sclerosis was decreased, and renal fibrosis was reduced even when tubular damage was quantified, allowing mesenchymal stem cells to have the therapeutic effect on renal fibrosis.

Furthermore, the present inventors confirmed that when renal fibrosis progressed in mice administered with adriamycin, the expression levels of various chemokines and CHI3L1 were changed (increased or reduced) and were regulated by the administration of mesenchymal stem cells. Typically, it was confirmed that CCL2, CCL4, CCL7, CCL11, CCL17, CCL19, CXCL9, CHI3L1, CXCL11, CXCL16, CX3CL1, CCL5, CCL8, and CCL20 were reduced.

Through this, it was confirmed that in order to predict the therapeutic effect of mesenchymal stem cells on renal fibrosis, CCL2, CCL4, CCL7, CCL11, CCL17, CCL19, CXCL9, CHI3L1, CXCL11, CXCL16, CX3CL1, CCL5, CCL8, and CCL20 may be utilized along with the existing biomarkers such as survival rate, the appearance of the kidney, the weight of the kidney, proteinuria and creatinine in blood, the size of the glomerulus, glomerular sclerosis level, and tubular damage level.

### Solution to Problem

The present invention may provide a biomarker composition for predicting the therapeutic effect of mesenchymal stem cells on renal fibrosis, comprising an agent for measuring an expression level of the mRNA or protein of CCL2, CCL4, CCL7, CCL17, CCL19, CXCL9, CXCL10, CXCL11, or CHI3L1.

The agent for measuring the mRNA expression level may be a primer pair, a probe or an antisense nucleotide that specifically binds to the mRNA.

The agent for measuring the protein expression level may be an antibody that specifically binds to the protein or a fragment of the protein.

The present invention may also provide a kit for predicting the therapeutic effect of mesenchymal stem cells on renal fibrosis, comprising the composition described above.

The kit may be a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

The present invention may also provide a method of providing information for predicting the therapeutic effect of mesenchymal stem cells on renal fibrosis, comprising: (a) measuring an expression level of the mRNA or protein of CCL2, CCL4, CCL7, CCL17, CCL19, CXCL9, CXCL10, CXCL11, or CHI3L1 from a sample; and (b) comparing the measured expression level to the expression level of a control group.

The sample may be obtained from a patient with renal fibrosis who has been treated with mesenchymal stem cells.

The measurement of the mRNA expression level may use reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology.

The measurement of the protein expression level may use Western blotting, immunohistochemical staining, immunoprecipitation assay, complement fixation assay or immunofluorescence.

The method may determine that the mesenchymal stem cells have the therapeutic effect or the excellent therapeutic effect when the gene expression level of CCL2, CCL4, CCL7, CCL17, CCL19, CXCL9, CXCL10, CXCL11, or CHI3L1 is measured to be low compared to that of the control sample.

### Effects of Invention

According to the present invention, CCL2, CCL4, CCL7, CCL17, CCL19, CXCL9, CXCL10, CXCL11, or CHI3L1 may be utilized to predict the therapeutic effect of mesenchymal stem cells on renal fibrosis.

### Brief Description of Drawings

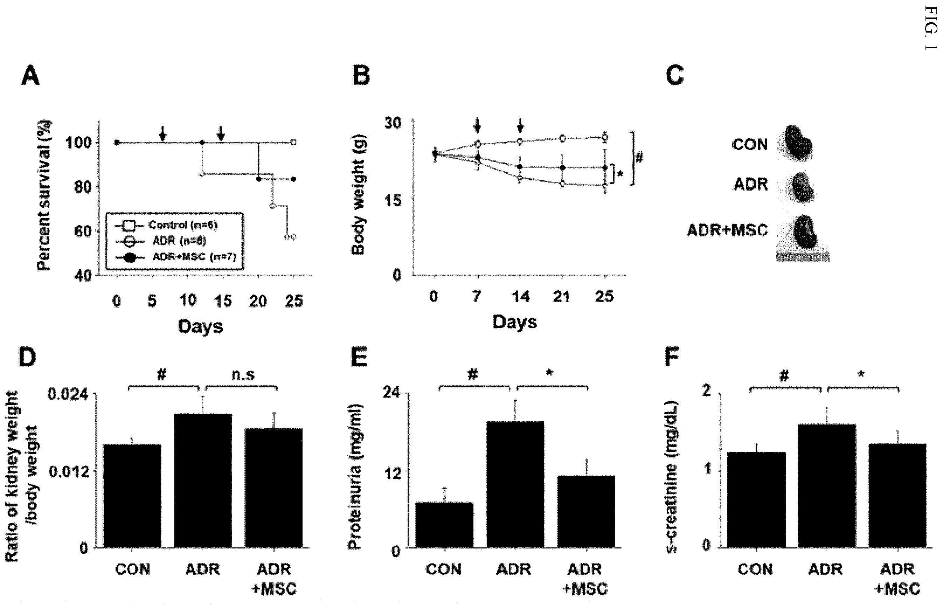

FIG. 1 illustrates a result obtained by confirming the effectiveness of mesenchymal stem cells in the renal fibrosis model by ADR. BALB/c mice were administered with ADR at 10 mg/kg into the tail vein, and 1 week and 2 weeks later, mesenchymal stem cells were administered into the tail vein at 1×10⁶ cells/mouse. (a) is a result obtained by measuring the survival rate for 4 weeks, (b) is a result obtained by measuring the body weight for 4 weeks, (c) is a result obtained by measuring the shape of the kidney by performing an autopsy at 4 weeks after ADR administration, (d) is a result obtained by measuring the weight of the kidney, (e) is a result obtained by measuring proteinuria in urine upon an autopsy, and (f) is a result obtained by measuring creatinine in serum.

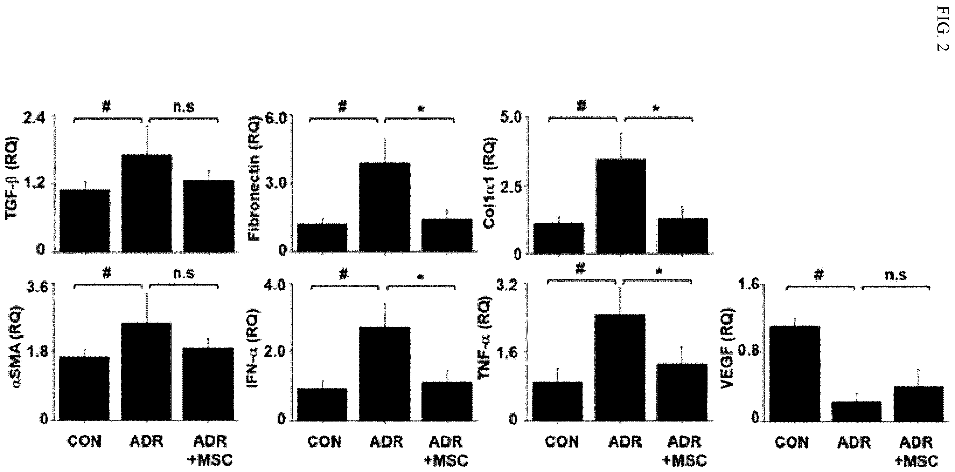

FIG. 2 illustrates a result obtained by confirming the expression of the fibrosis marker gene in the kidney. It is a result obtained by isolating the RNA in the kidney at 4 weeks after ADR administration and measuring the expression level of the fibrosis marker gene by qPCR for gene analysis for the fibrosis marker.

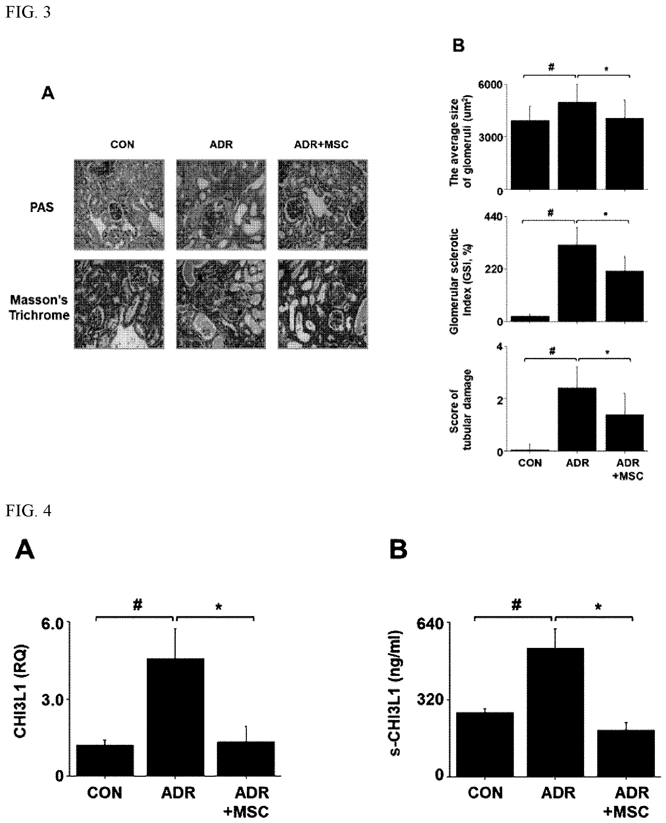

FIG. 3 illustrates a result obtained by confirming the histopathological changes in the kidney. Histopathological examination was performed in the kidney at 4 weeks after ADR administration. (a) is a result obtained by confirming the changes in glomerulus, basement membrane and tubule, and collagen production by staining with PAS and Masson's trichrome, and (b) is a result obtained by quantifying the size of the glomerulus, glomerular sclerosis level, and tubular damage by analyzing 40 fields per each group.
FIG. 4 illustrates a result obtained by measuring CHI3L1 in the renal fibrosis model. It is a result obtained by isolating the kidney and serum at 4 weeks after ADR administration, isolating RNA from the kidney, and measuring the gene expression of CHI3L1 through qPCR. (a) is a result obtained by calculating the relative quantitative value based on a control group, and (b) is a result obtained by measuring the amount of CHI3L1 in the serum through ELISA.

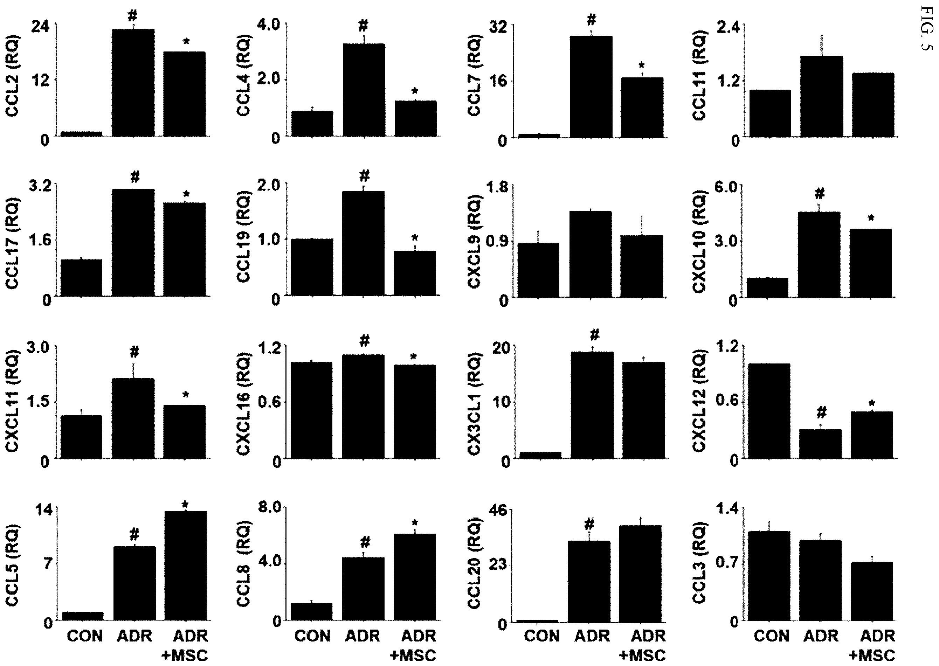

FIG. 5 illustrates a result obtained by confirming the chemokine changes in the kidney in the renal fibrosis model. It is a result obtained by isolating the kidney at 4 weeks after ADR administration, isolating RNA from the tissue, performing qPCR for chemokines, and calculating as a relative quantitative value for each chemokine based on a control group.

### Mode for Carrying out the Invention

Hereinafter, the present invention will be described in more detail.

The present inventors measured the therapeutic effect of mesenchymal stem cells in a renal fibrosis disease animal model using the existing biomarkers (survival rate, the appearance of the kidney, the weight of the kidney, proteinuria and creatinine in blood, the size of the glomerulus, glomerular sclerosis level, and tubular damage), and confirmed that new biomarkers (chemokines and CHI3L1) can be also measured. Based on the above, the present inventors completed the present invention.

The present invention may provide a biomarker composition for predicting the therapeutic effect of mesenchymal stem cells on renal fibrosis, comprising an agent for measuring an expression level of the mRNA or protein of CCL2, CCL4, CCL7, CCL17, CCL19, CXCL9, CXCL10, CXCL11, or CHI3L1.

The present inventors confirmed that administration of adriamycin leads to renal fibrosis in mice, which leads to renal fibrosis, turns hard and white, increases in weight, increases proteinuria levels, increases blood creatinine concentration, increases renal fibrosis, and increases the expression of EMT markers and inflammatory cytokines, and the glomerulus is hardened due to increased collagen production, and the tubules are contracted and eventually leads to death. When mesenchymal stem cells were administered to these mice, these mice showed a similar renal status to that of the control group in which renal fibrosis was not induced, decreased kidney weight, decreased proteinuria level and blood creatinine concentration, decreased renal fibrosis, the decreased expression of EMT markers and inflammatory cytokines, and increased VEGF. Histopathologically, it was confirmed that collagen production in the glomerulus was decreased, sclerosis was decreased, and renal fibrosis was reduced even when tubular damage was quantified, allowing mesenchymal stem cells to have the therapeutic effect on renal fibrosis.

Furthermore, the present inventors confirmed that when renal fibrosis progressed in mice administered with adriamycin, the expression levels of various chemokines and CHI3L1 were changed (increased or reduced) and were regulated by the administration of mesenchymal stem cells. Typically, it was confirmed that CCL2, CCL4, CCL7, CCL11, CCL17, CCL19, CXCL9, CHI3L1, CXCL11, CXCL16, CX3CL1, CCL5, CCL8, and CCL20 were reduced.

Through this, it was confirmed that in order to predict the therapeutic effect of mesenchymal stem cells on renal fibrosis, CCL2, CCL4, CCL7, CCL11, CCL17, CCL19, CXCL9, CHI3L1, CXCL11, CXCL16, CX3CL1, CCL5, CCL8, and CCL20 may be utilized along with the existing biomarkers such as survival rate, the appearance of the kidney, the weight of the kidney, proteinuria and creatinine in blood, the size of the glomerulus, glomerular sclerosis level, and tubular damage level.

As used herein, the term "prediction" means presuming to guess about a medical consequence. For the purpose of the present invention, it means presuming the course of the disease (disease progression, improvement, recurrence of renal fibrosis, tumor growth, drug resistance) of a patient diagnosed with renal fibrosis.

As used herein, the term "agent for measuring an expression level of the mRNA or protein" refers to a molecule that may be used for detection and/or quantification of a marker by determining an expression level of the mRNA or protein of CCL2, CCL4, CCL7, CCL17, CCL19, CXCL9, CXCL10, CXCL11, or CHI3L1, which is a marker that decreases after treatment with mesenchymal stem cells in a patient with renal fibrosis. Specifically, the agent for measuring the mRNA expression level of the gene may be a primer pair, a probe or an antisense nucleotide that specifically binds to the mRNA. In this regard, since the nucleic acid information of the genes is known in genebank, etc., those of ordinary skill in the art may design a primer or a probe that specifically amplifies a specific region of these genes based on the sequence.

As used herein, the term "primer pair" includes all combinations of primer pairs consisting of forward and reverse primers recognizing a target gene sequence, and specifically, a primer pair that provides analysis results with specificity and sensitivity. Since the nucleic acid sequence of the primer is a sequence that is inconsistent with a non-target sequence present in the sample, high specificity may be conferred when the primer amplifies only the target gene sequence containing the complementary primer binding site and does not cause non-specific amplification.

As used herein, the term "probe" refers to a material capable of specifically binding to a target material to be detected in a sample, and refers to a material capable of specifically confirming the presence of a target material in the sample through the binding. The type of a probe molecule is not limited as a material commonly used in the art, but it may be preferably PNA (peptide nucleic acid), LNA (locked nucleic acid), peptide, polypeptide, protein, RNA or DNA. More specifically, the probe is a biomaterial derived from an organism or similar thereto, or includes one manufactured ex vivo. For example, the probe may be enzymes, proteins, antibodies, microorganisms, animal and plant cells and organs, nerve cells, DNA, and RNA, and DNA includes cDNA, genomic DNA, and oligonucleotides, RNA includes genomic RNA, mRNA, and oligonucleotides, and examples of proteins may include antibodies, antigens, enzymes, peptides, and the like.

As used herein, the term "antisense oligonucleotide" refers to DNA or RNA or a derivative thereof containing a nucleic acid sequence complementary to a specific mRNA sequence, which binds to a complementary sequence in mRNA and acts to inhibit the translation of mRNA into protein. An antisense oligonucleotide sequence refers to a DNA or RNA sequence that is complementary to mRNA of the genes and is capable of binding to the mRNA. This may inhibit the translation, translocation into the cytoplasm, maturation or essential activity for all other overall biological functions of the gene mRNA. The length of the antisense oligonucleotide may be 6 to 100 base pair (bp), preferably 8 to 60 bp, more preferably 10 to 40 bp. The antisense oligonucleotide may be synthesized in vitro by a conventional method and administered in vivo, or the antisense oligonucleotide may be synthesized in vivo. One example of synthesizing an antisense oligonucleotide in vitro is to use RNA polymerase I. One example of synthesizing an antisense RNA in vivo is to transcribe an antisense RNA by using a vector in which the origin of a multiple cloning site (MCS) is in the opposite direction. Preferably, the antisense RNA is not translated into a peptide sequence by allowing a translation stop codon to be present in the sequence.

The agent for measuring the protein expression level may be an antibody that specifically binds to the protein or a fragment of the protein, and the antibody may be a polyclonal antibody or a monoclonal antibody.

As used herein, the term "antibody" may refer to a specific protein molecule directed against an antigenic region. For the purposes of the present invention, an antibody refers to an antibody that specifically binds to a marker protein, and includes a polyclonal antibody, a monoclonal antibody and a recombinant antibody. An antibody may be readily prepared using techniques well known in the art. In addition, the antibody of the present specification includes a complete form having two full length light chains and two full length heavy chains, as well as functional fragments of the antibody molecule. A functional fragment of the antibody molecule refers to a fragment having at least an antigen binding function, and includes Fab, F (ab'), F (ab')₂ and Fv, etc.

The present invention may also provide a kit for predicting the therapeutic effect on renal fibrosis, comprising the composition described above. Specific details of the composition are as described above. The kit may predict the therapeutic effect of mesenchymal stem cells on renal fibrosis by measuring the protein expression level of CCL2, CCL4, CCL7, CCL17, CCL19, CXCL9, CXCL10, CXCL11, or CHI3L1, which is a marker for predicting the therapeutic effect of mesenchymal stem cells on renal fibrosis, or an expression level of the mRNA or protein of the gene. The kit may comprise an agent for measuring the mRNA expression level of a marker gene for predicting the therapeutic effect of mesenchymal stem cells on renal fibrosis, for example, a primer pair, a probe or an antisense nucleotide that specifically binds to the gene, and may comprise an agent for measuring the protein expression level, for example, an antibody that specifically binds to the marker protein or an antigen binding fragment of the antibody.

The kit may be a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

The kit for predicting the therapeutic effect of mesenchymal stem cells on renal fibrosis may further include one or more other component compositions, solutions or devices suitable for the analysis method. For example, the diagnostic kit may be a diagnostic kit comprising essential elements necessary for performing a reverse transcription polymerase chain reaction. The reverse transcription polymerase chain reaction kit includes each primer pair specific for a marker gene. The primer is a nucleotide having a sequence specific to the nucleic acid sequence of each gene, and has a length of about 7 bp to 50 bp, more preferably a length of about 10 bp to 30 bp. In addition, it may include a primer specific for the nucleic acid sequence of a control gene. In addition, the reverse transcription polymerase chain reaction kit may include test tubes or other proper containers, reaction buffers (varying pH and magnesium concentrations), deoxynucleotides (dNTPs), enzymes such as Taq-polymerase and reverse transcriptase, DNAse, RNAse inhibitors, DEPC-water, sterile water, and the like. In addition, for example, the DNA chip kit may include a substrate to which cDNA or oligonucleotide corresponding to a gene or a fragment thereof is attached, and reagents, agents and enzymes for preparing a fluorescently labeled probe, and the like. In addition, substrate may include cDNA or oligonucleotide corresponding to a control gene or a fragment thereof. In addition, for example, the kit may be a diagnostic kit comprising essential elements necessary for performing ELISA. The ELISA kit includes an antibody specific for the protein. Antibodies may be antibodies having high specificity and affinity for each marker protein and little cross-reactivity with other proteins, and may be monoclonal antibodies, polyclonal antibodies or recombinant antibodies. In addition, the ELISA kit may include an antibody specific for a control protein. In addition, the ELISA kit may include reagents capable of detecting bound antibodies, for example, labeled secondary antibodies, chromophores, enzymes (for example, conjugated with an antibody) and substrates thereof or other materials capable of binding an antibody and the like. In addition, for example, the kit may be a rapid kit comprising essential elements necessary for performing a rapid assay that may determine the analysis result. The rapid kit includes an antibody specific for the protein. Antibodies may be antibodies having high specificity and affinity for each marker protein and little cross-reactivity with other proteins, and may be monoclonal antibodies, polyclonal antibodies or recombinant antibodies. In addition, the rapid kit may include an antibody specific for a control protein. In addition, the rapid kit may include reagents capable of detecting bound antibody, for example, a nitrocellulose membrane to which a specific antibody and a secondary antibody are immobilized, a membrane bound to an antibody bound bead, other materials such as an absorbent pad and a sample pad, and the like. In addition, for example, the kit may be an MRM (multiple reaction monitoring) kit in MS/MS mode comprising essential elements necessary for performing mass spectrometry. While SIM (selected ion monitoring) is a method that uses ions generated by one collision at the source part of the mass spectrometer, MRM is a method of selecting a specific ion from among the broken ions once more, passing it through another continuously connected MS source once more, colliding it to obtain the ions, and then using the ions among them. The MRM (multiple reaction monitoring) analysis methods may compare the protein expression level of the normal control group or the same subject with the protein expression level of a subject who has been treated with mesenchymal stem cells, and may also predict the therapeutic effect of mesenchymal stem cells on renal fibrosis by determining whether there is a significant increase or decrease in the expression level from the gene into the protein in a marker for predicting the therapeutic effect of mesenchymal stem cells on renal fibrosis.

The present invention may also provide a method of providing information useful for predicting the therapeutic effect of mesenchymal stem cells on renal fibrosis, comprising: (a) measuring an expression level of the mRNA or protein of CCL2, CCL4, CCL7, CCL17, CCL19, CXCL9, CXCL10, CXCL11, or CHI3L1 from a sample; and (b) comparing the measured expression level to the expression level of a control group.

The sample may be obtained from a patient with renal fibrosis who has been treated with mesenchymal stem cells.

The method of providing the information comprises measuring an expression level of the mRNA or protein of CCL2, CCL4, CCL7, CCL17, CCL19, CXCL9, CXCL10, CXCL11, or CHI3L1 in a biological sample isolated from a subject. Specifically, the expression level of mRNA or protein may be determined by measuring the mRNA expression level of the marker gene or the expression level of the protein encoded by the gene. Isolation of mRNA or protein from a sample of a subject may be appropriately performed by those of ordinary skill in the art according to methods known in the art. For example, after homogenizing the kidney tissue of a subject with a buffer for extracting protein or a buffer for extracting nucleic acid, and then centrifuging it, the obtained supernatant may be used as a sample for the subject. The subject may include a vertebrate, a mammal, or a human (Homo sapiens). The sample may be any one or more selected from the group consisting of tissue, cells, whole blood, serum and plasma.

The measurement of the mRNA expression level may use reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology.

The measurement of the protein expression level may use Western blotting, immunohistochemical staining, immunoprecipitation assay, complement fixation assay or immunofluorescence.

The method may determine that the mesenchymal stem cells have the therapeutic effect or the excellent therapeutic effect when the gene expression level of CCL2, CCL4, CCL7, CCL17, CCL19, CXCL9, CXCL10, CXCL11, or CHI3L1 is measured to be low compared to that of the control sample.

Hereinafter, the present invention will be described in more detail through examples. The following examples are only preferred specific examples of the present invention, and the scope of the present invention is not limited to the scope of the following examples.

### <Example 1> Design of animal experiment

Adriamycin (ADR) generates hydroxyl radicals, hydrogen peroxide, and superoxide anions to induce fat peroxidation in glomerular epithelial cells and generate toxicity response of podocytes and inflammatory response (T cell, B cell, and macrophage influx). A single administration of ADR at 10 mg/kg to the tail vein of the mice induced proteinuria, glomerulus and podocyte lesions. This causes the glomerular canal to shrink and eventually fibrosis. This phenomenon may represent MCD (minimal change disease), FSGS (focal segmental glomerulosclerosis), nephrotic syndrome, and glomerulosclerosis in humans. Male BALB/c mice were purchased from Samtako. The breeding environment of the mice was maintained at a temperature of 21 ~ 24 °C, a humidity of 40 ~ 60%, and a light/dark cycle of 12 hours. All experimental animals were acclimatized in the animal room for 1 week before use. All animal experiments were approved by the Animal Experimental Ethics Committee of Chungbuk National University and were performed according to approved guidelines. Human derived mesenchymal stem cells (human MSC) were purchased from CORESTEM, Inc. Normal bone marrow derived mesenchymal stem cells were cultured using CSBM-A06 medium, and passage 4 (p4) cells were used for the experiment. 9-week-old male BALB/c mice were administered with ADR at 10 mg/kg into the tail vein, and then 1 week and 2 weeks later, human derived mesenchymal stem cells were administered at 1×10⁶ cells/mouse into the tail vein. As shown in FIGs. 1A and 1B, as a result of administering ADR to BALB/c mice and measuring the survival rate and weight for 4 weeks, the group administered with ADR only showed a survival rate of 57.1%, whereas the group administered with mesenchymal stem cells after ADR administration showed a survival rate of 83.3%. In addition, the group administered with mesenchymal stem cells did not show a significant decrease in mouse weight compared to the group administered with ADR only. 4 weeks after ADR administration, an autopsy was performed, and urine, serum, and kidneys were separated and analyzed. Compared to the control group, the kidneys of the group administered with ADR turned hard and white in color due to fibrosis, whereas the group administered with mesenchymal stem cells showed a renal status similar to that of the control group (FIG. 1C). When the weight of the kidney compared to the weight of the mouse was measured, the group administered with ADR showed the increased weight of the kidney compared to the control group, and showed a tendency to decrease when mesenchymal stem cells were administered (FIG. 1D).

### <Example 2> Measurement of proteinuria

Proteinuria was measured using the urine isolated from the animal model of Example 1, and it was performed according to the test method provided by Pierce 660 nm Protein Assay (Thermo Fisher Scientific). The mouse urine was diluted with PBS, and then 10 µl of the diluted sample was reacted with 150 µl of protein assay reagent, and 10 minutes later, the OD value was measured at 660 nm. When the kidneys become fibrous, the filtering function to filter metabolites and wastes is broken, and protein is detected in the urine. Accordingly, as a result of measuring proteinuria using the urine, it was confirmed that proteinuria was increased in the group administered with ADR compared to the control group, whereas it was decreased when mesenchymal stem cells were administered (FIG. 1E).

### <Example 3> Measurement of creatinine

Creatinine was measured using the serum isolated from the animal model of Example 1, and it was performed according to the test method provided by Serum Creatinine ELISA kit (ALPHA DIAGNOSTIC INTERNATIONAL). The mouse serum was diluted with DW, and then 25 µl of the diluted sample was reacted with 25 µl of assay diluent and 100 µl of creatinine reagent, and the OD value was measured at 490 nm after 1 minute and 30 minutes, respectively. When the kidneys become fibrous, the filtering function to filter metabolites and wastes is broken, and creatinine is increased in the serum. Accordingly, as a result of measuring creatinine using the serum, it was confirmed that the creatinine concentration was increased in the group administered with ADR compared to the control group, whereas it was decreased when mesenchymal stem cells were administered (FIG. 1F). Therefore, it was confirmed that mesenchymal stem cells had the therapeutic effect in the ADR-induced renal fibrosis model.

### <Example 4> Quantitative real-time PCR (qPCR)

By measuring various cytokines in the kidney, the therapeutic effect of mesenchymal stem cells on renal fibrosis was determined. Total RNA was isolated from the kidney tissue using TRIZOL Reagent (Invitrogen). cDNA was synthesized at a concentration of 1 µg of total RNA. qPCR was performed on the synthesized cDNA using a SYBR green probe at a concentration of 50 ng. TGF-β, fibronectin, and Col1α1 (Collagen alpha-1) are fibrosis markers, and α-SMA is a marker of epithelial-mesenchymal transition (EMT) in which epithelial cells are converted into mesenchymal stem cells and cell migration and invasion are increased. IFN-α and TNF-α are cytokines associated with the inflammatory response, and VEGF may be identified as a survival factor of podocytes constituting the kidney. As can be seen in FIG. 2, renal fibrosis, the expression of EMT markers and inflammatory cytokines were increased in the group administered with ADR compared to the control group and were decreased when mesenchymal stem cells were administered. In addition, it was confirmed that when ADR was administered, the kidneys were fibrotic and the podocytes were damaged, resulting in a decrease in VEGF, but an increase in VEGF when mesenchymal stem cells were administered.

### <Example 5> Preparation of tissue sample and histopathology

Histopathological changes in the kidneys were confirmed at 4 weeks after ADR administration. When the kidneys become fibrous, the glomerulus changes into an abnormal shape, the tubules contract, collagen is formed, and inflammatory cells are increased. In addition, the components of the basement membrane and capillaries are thickened. These histological changes in the kidneys can be identified by staining with PAS and Masson's trichrome. PAS is a representative test method used to differentiate the changes in glomerular lesions and Bowman's capsule by oxidizing polysaccharides, a component of the basement membrane, with periodic acid, and displaying the glomerular basement membrane in red. Masson's trichrome is a test method used to confirm the histological changes in collagen production due to renal fibrosis. Kidneys were fixed in formaldehyde solution, and paraffin blocks were prepared according to standard tissue sample preparation methods. The tissue was sectioned to a thickness of 4 µm and stained with Periodic acid-Schiff (PAS) and Masson's trichrome. Using the PAS-stained tissue, the size of the glomerulus was measured through the AxioVision program. The glomerular sclerotic index was determined on the basis of glomerular sclerosis, and the degree of these changes was scored (0, none; 1, < 25% sclerosis observed; 2, 25~49% sclerosis observed; 3, 50~74% sclerosis observed; 4, <75% sclerosis observed) (American Journal of the Medical Sciences, 2013;346 (6):486-493). The tubular damages were scored (0, none; 1, tubules very slightly partially dilated; 2, a large number of tubules dilated and interstitial dilation; 3, tubules very excessively dilated and interstitial cyst formed; 4, tubules contracted) (Kidney Int. 1987;31:718-724, J Hypertension 1991;9:719-728). In the group administered with ADR compared to the control group, it was confirmed that the glomerulus was enlarged and the glomerular lesion was induced during PAS staining, which resulted in the increased redness of the basement membrane and the contracted tubules. In addition, it was confirmed that the blueness of the stained collagen was increased through Masson's trichrome staining. However, when mesenchymal stem cells were administered, glomerular changes and collagen production were decreased (FIG. 3A). It was confirmed that renal fibrosis was decreased in the group administered with mesenchymal stem cells compared to the group administered with ADR even when the size of the glomerulus, glomerular sclerosis level, and tubular damage were quantified based on histological changes (FIG. 3B).

### <Example 6> ELISA measurement of CHI3L1

CHI3L1 (Chitinase-3-like protein1, Ym-1), known as YKL-40 in humans, is a 40 KDa glycoprotein that is present in the form in which amino acids are present at three N-terminal ends. It is expressed in macrophages, fibroblasts, pseudosynovial cells, vascular smooth muscle cells, and hepatic gland cells, and the like, and does not have the hydrolytic function of chitin, which is the original role of chitinase. Although the exact biological mechanism of CHI3L1 is not known, it is known as an inflammatory marker for inflammatory response, extracellular tissue remodeling, fibroma, solid cancer, and asthma, and the like. In particular, it has been reported that it is involved in Th2 inflammatory response in cancer, increases anti-apoptotic activity, and increases angiogenesis to induce cancer cell proliferation. As such, by confirming whether CHI3L1, which is associated with inflammation and induction of cell proliferation, also acts on renal fibrosis, we examined whether it can be applied as a biomarker for predicting the onset and the therapeutic effect by mesenchymal stem cells. CHI3L1 was measured using the isolated serum and it was performed according to the test method provided by Mouse Chitinase 3-like 1 ELISA kit (R&D SYSTEM). Serum was diluted 500-fold with assay diluent before use. It was confirmed that the gene expression of CHI3L1 in the kidney (FIG. 4A) and the production of CHI3L1 in the serum (FIG. 4B) were increased in the group administered with ADR compared to the control group, and the gene expression of CHI3L1 and the production of CHI3L1 in the serum were statistically significantly decreased when mesenchymal stem cells were administered. Therefore, it was confirmed that CHI3L1 could be utilized as a biomarker for predicting the onset of renal fibrosis and the therapeutic effect by mesenchymal stem cells.

### <Example 7> Measurement of chemokine

Chemokines are cytokines involved in cell migration, and are small-molecular proteins involved in the movement of lymphocytes, and the formation, maintenance, and development of lymphoid tissues in the inflammatory response. When the kidney is inflamed, cells in the kidney secrete chemokines, and the inflammatory immune cells further invade the kidney, resulting in tissue damage and loss of function. Therefore, if chemokines are measured, the degree of inflammatory response in the kidneys can be confirmed.

The gene expression of the inflammatory chemokines CCL2, CCL4, CCL7, CCL11, CCL17, CCL19, CXCL9, CXCL10, CXCL11, CXCL16, CX3CL1, CXCL12, CCL5, CCL8, CCL20, and CHI3L1 was measured using TRIZOL Reagent (Invitrogen). cDNA was synthesized at a concentration of 1 µg of total RNA. qPCR was performed on the synthesized cDNA using a SYBR green probe at a concentration of 50 ng.

As can be seen in FIG. 5, administration of ADR in the renal fibrosis model increased the chemokines CCL2, CCL4, CCL7, CCL11, CCL17, CCL19, CXCL9, CHI3L1, CXCL11, CXCL16, CX3CL1, CCL5, CCL8, and CCL20. Therefore, CCL2, CCL4, CCL7, CCL17, CCL19, CXCL9, CXCL10, CXCL11, and CHI3L1 had a tendency to decrease when mesenchymal stem cells were administered, and thus, CCL2, CCL4, CCL7, CCL17, CCL19, CXCL9, CXCL10, CXCL11, and CHI3L1 can be used as a diagnostic marker useful for confirming renal fibrosis and predicting the effectiveness of mesenchymal stem cells.

## Claims

1. A biomarker composition for predicting the therapeutic effect of mesenchymal stem cells on renal fibrosis, comprising an agent for measuring an expression level of the mRNA or protein of CCL2, CCL4, CCL7, CCL17, CCL19, CXCL9, CXCL10, CXCL11, or CHI3L1.

2. The biomarker composition for predicting the therapeutic effect of mesenchymal stem cells on renal fibrosis according to claim 1, wherein the agent for measuring the mRNA expression level is a primer pair, a probe or an antisense nucleotide that specifically binds to the mRNA.

3. The biomarker composition for predicting the therapeutic effect of mesenchymal stem cells on renal fibrosis according to claim 1, wherein the agent for measuring the protein expression level is an antibody that specifically binds to the protein or a fragment of the protein.

4. A kit for predicting the therapeutic effect of mesenchymal stem cells on renal fibrosis, comprising the composition of claim 1.

5. The kit for predicting the therapeutic effect of mesenchymal stem cells on renal fibrosis according to claim 4, wherein the kit is a RT-PCR (reverse transcription polymerase chain reaction) kit, a DNA chip kit, an ELISA (enzyme-linked immunosorbent assay) kit, a protein chip kit, a rapid kit or an MRM (multiple reaction monitoring) kit.

6. A method of providing information for predicting the therapeutic effect of mesenchymal stem cells on renal fibrosis, comprising:
measuring an expression level of the mRNA or protein of CCL2, CCL4, CCL7, CCL17, CCL19, CXCL9, CXCL10, CXCL11, or CHI3L1 from a sample; and
comparing the measured expression level to the expression level of a control group.

7. The method of providing information for predicting the therapeutic effect of mesenchymal stem cells on renal fibrosis according to claim 6, wherein the measurement of the mRNA expression level uses reverse transcription polymerase chain reaction (RT-PCR), competitive reverse transcription polymerase chain reaction (competitive RT-PCR), real time reverse transcription polymerase chain reaction (real time quantitative RT-PCR), quantitative polymerase chain reaction (quantitative RT-PCR), RNase protection method, Northern blotting or DNA chip technology.

8. The method of providing information for predicting the therapeutic effect of mesenchymal stem cells on renal fibrosis according to claim 6, wherein the measurement of the protein expression level uses Western blotting, immunohistochemical staining, immunoprecipitation assay, complement fixation assay or immunofluorescence.

9. The method of providing information for predicting the therapeutic effect of mesenchymal stem cells on renal fibrosis according to claim 6, wherein the method determines that the mesenchymal stem cells have the therapeutic effect or the excellent therapeutic effect when the gene expression level of CCL2, CCL4, CCL7, CCL17, CCL19, CXCL9, CXCL10, CXCL11, or CHI3L1 is measured to be low compared to that of the control sample.
